# EUROPEAN PATENT APPLICATION

(11) **EP 1 849 765 A1**
(43) Date of publication of application: **31.10.2007**
(21) Application number: 06713355.3
(22) Date of filing: 02.02.2006
(51) Int. Cl.: C07C 67/31, C07D 239/38, C07C 69/738, C07D 239/42

(54) **METHOD FOR PRODUCING PYRIMIDINE COMPOUND**

(30) Priority: 02.02.2005 JP 2005027065
(71) Applicant: Ajinomoto Co., Inc., Tokyo 104-8315 (JP)
(72) Inventor: TAKAHASHI, Daisuke c/o AJINOMOTO CO., INC., Kanagawa, 2108681 (JP); IZAWA, Kunisuke c/o AJINOMOTO CO., INC., Kanagawa, 21086 (JP)
(74) Representative: Nicholls, Kathryn Margaret
(86) International application number: PCT/JP2006/302212
(87) International publication number: WO 2006/083012

(57) **Abstract**

Compound (II) and compound (III) are reacted to give compound (IVa) and/or compound (IVb); which are/is then reacted with compound (V) to give compound (I). 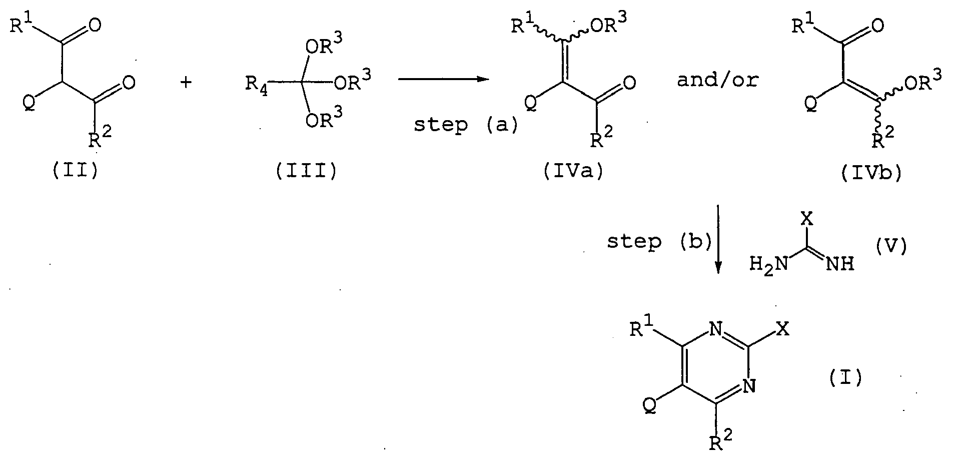 wherein X is a methylthio group and the like, R¹ and R² are each a lower alkyl group optionally having substituent(s), an aryl group optionally having substituent(s) and the like, R³ is a lower alkyl group, R⁴ is a hydrogen atom, a lower alkyl group optionally having substituent(s) and the like, and Q is a carboxylate group and the like.

## Description

### Technical Field

The present invention relates to a production method of a pyrimidine compound useful as an intermediate for synthesizing a pharmaceutical product.

### Background Art

A pyrimidine compound represented by the formula (I): 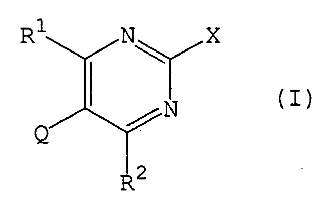 wherein R¹ and R² are the same or different and each is a lower alkyl group optionally having substituent(s), an aralkyl group optionally having substituent(s) or an aryl group optionally having substituent(s), Q is a carboxylate group and X is a hydrogen atom, a lower alkyl group optionally having substituent(s), an aralkyl group optionally having substituent(s), an aryl group optionally having substituent(s), -OR⁵, -SR⁵ or -NR⁵R⁶ (wherein R⁵and R⁶ are the same or different and each is a hydrogen atom, a lower alkyl group optionally having substituent(s), an aralkyl group optionally having substituent(s), an aryl group optionally having substituent(s) or -SO₂R⁷ (wherein R⁷ is a lower alkyl group optionally having substituent(s) or an aryl group optionally having substituent(s)), or R⁵ and R⁶ may form, together with the adjacent nitrogen atom, an aliphatic hetero ring optionally having substituent(s)), is useful as a versatile synthetic intermediate for various pharmaceutical products. For example, HMGCoA reductase inhibitors (EP 0022478 and JP-A-5-178841), anti-HIV drugs (JP-A-2004-508421), calcium channel antagonists (WO02/022588) and the like can be mentioned.

As the production method of the pyrimidine compound, a method shown in the following scheme, which comprises reacting cinnamic acid derivative (1) with methylisothiourea to give dihydropyrimidine derivative (2), and dehydrogenating (2) to give pyrimidine compound (3) is known (WO02/022588)

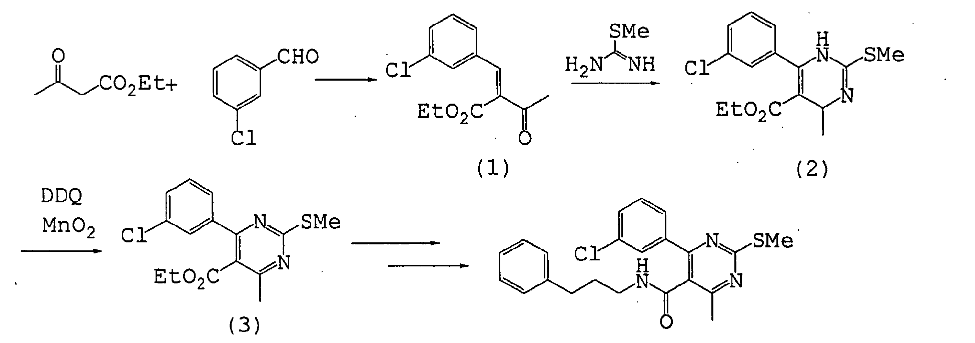

In this method, however, when 2,3-dichloro-5,6-dichloro-p-benzoquinone (DDQ) is used for dehydrogenation, the cost increases since DDQ is expensive, and when manganese dioxide is used for dehydrogenation, heavy metal wastes requiring processing are produced. As mentioned above, various problems occur due to dehydrogenation and, moreover, the yield is not satisfactory.

As other method, a method shown in the following scheme, which comprises leading benzoylacetate (4) to enol phosphate (5), and reacting (5) with methylisothiourea or methylguanidine has been reported (JP-A-6-256318). 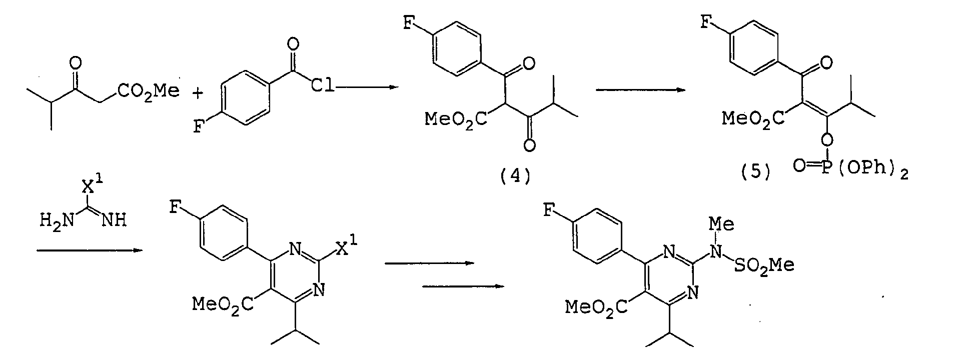 wherein X¹ is a methylthio group or a methylamino group.

According to this method, however, the phosphorylation reagent to be used is expensive, and the yield is 20 - 55%, which is not satisfactory. The method is also associated with the problems of difficult removal, from the desired product, of a phosphorylation reagent remaining after the conversion to enol phosphorate (5) and a phosphoric acid derivative eliminated during cyclization.

As still another method, a method shown in the following scheme, which comprises converting methyl diacetylacetate (6) to methyl enol ether (7) using methyl trifluoromethanesulfonate and cesium carbonate, and reacting (7) with formamidine is described (Bioorganic & Medicinal Chemistry Letters, 2003, vol. 13, No. 4, p.709-712).

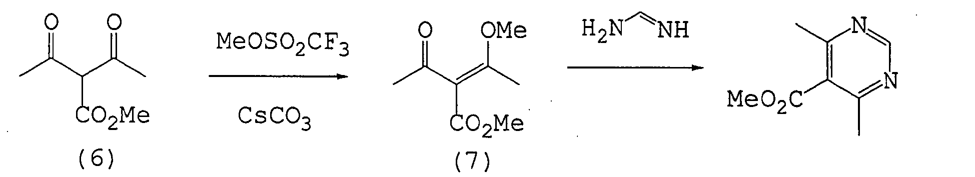

However, the method is associated with problems in that the cost increases since methyl trifluoromethanesulfonate and cesium carbonate used for enol etherification are expensive, methyl trifluoromethanesulfonate is highly toxic, which is unpreferable from the aspects of safety and hygiene, and a considerable amount of C-methyl form is byproduced during enol etherification.

### Disclosure of the Invention

An object of the present invention is to provide an industrially advantageous production method of a pyrimidine compound useful as a synthetic intermediate for a pharmaceutical product.

The present inventors have conducted intensive studies in an attempt to solve the above-mentioned problems and established a synthetic route permitting safe production of the pyrimidine compound at a low cost and in a high yield, which resulted in the completion of the present invention. Accordingly, the present invention provides the following [1] - [15].
[1] A production method of a compound represented by the following formula (IVa): 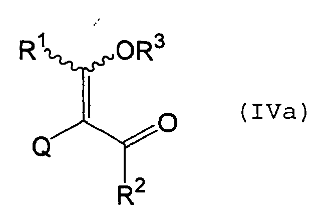 wherein R¹ and R² are the same or different and each is a lower alkyl group optionally having substituent(s), an aralkyl group optionally having substituent(s) or an aryl group optionally having substituent(s), R³ is a lower alkyl group, Q is a carboxylate group, and the wavy line is a trans-isomer, a cis-isomer or a mixture thereof (hereinafter sometimes to be referred to as compound (IVa)) and/or a compound represented by the formula (IVb): 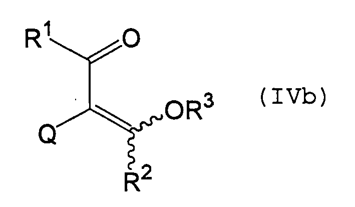 wherein the wavy line and each symbol are as defined above (hereinafter sometimes to be referred to as compound (IVb)), which comprises reacting a compound represented by the formula (II) : 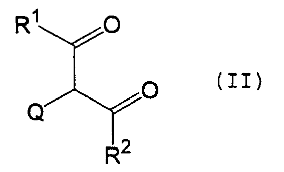 wherein each symbol is as defined above (hereinafter sometimes to be referred to as compound (II)) with an ortho ester represented by the formula (III): 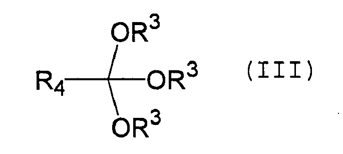 wherein R³ is as defined above, and R⁴ is a hydrogen atom, a lower alkyl group optionally having substituent(s) or an aryl group optionally having substituent(s) (hereinafter sometimes to be referred to as ortho ester (III)).
[2] A production method of a compound represented by the formula (I): 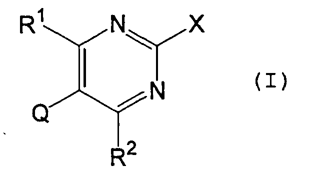 wherein X is a hydrogen atom, a lower alkyl group optionally having substituent(s), an aralkyl group optionally having substituent(s), an aryl group optionally having substituent(s), -OR⁵, -SR⁵ or -NR⁵R⁶ (wherein R⁵ and R⁶ are the same or different and each is a hydrogen atom, a lower alkyl group optionally having substituent(s), an aralkyl group optionally having substituent(s), an aryl group optionally having substituent(s) or -SO₂R⁷ (wherein R⁷ is a lower alkyl group optionally having substituent(s) or an aryl group optionally having substituent(s)), or R⁵ and R⁶ may form, together with the adjacent nitrogen atom, an aliphatic hetero ring optionally having substituent(s)), and other symbols are as defined above (hereinafter sometimes to be referred to as compound (I)) or a salt thereof, which comprises reacting compound (IVa) and/or compound (IVb) obtained by the production method of the above-mentioned [1], with a compound represented by the formula (V): or 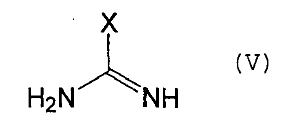 wherein X is as defined above (hereinafter sometimes to be referred to as compound (V)) or a salt thereof.
[3] The production method of the above-mentioned [2], wherein X is a hydrogen atom, a methyl group, a phenyl group, a methoxy group, a methylthio group, a benzylthio group, an amino group, a methylamino group or an N-methanesulfonyl-N-methylamino group.
[4] The production method of any one of the above-mentioned [1] to [3], wherein R¹ is a methyl group or an isopropyl group, and R² is a methyl group or an aryl group optionally having substituent(s).
[5] The production method of any one of the above-mentioned [1] to [4], wherein Q is a methoxycarbonyl group or an ethoxycarbonyl group.
[6] The production method of the above-mentioned [2], wherein X is a methylthio group, R¹ is an isopropyl group, and R² is a 4-fluorophenyl group.
[7] A production method of a compound represented by the formula (VII) : 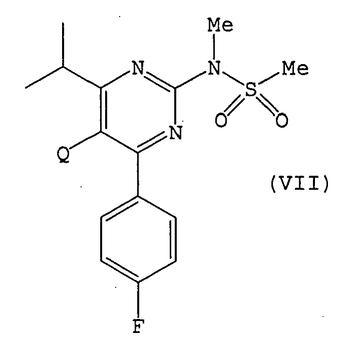 wherein Q is a carboxylate group (hereinafter sometimes to be referred to as compound (VII)) or a salt thereof, which comprises oxidizing a compound represented by the formula (I'): 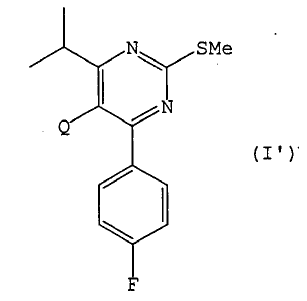 wherein Q is as defined above (hereinafter sometimes to be referred to as compound (I')), which is obtained by the production method of the above-mentioned [5], or a salt thereof to give a compound represented by the formula (VI): 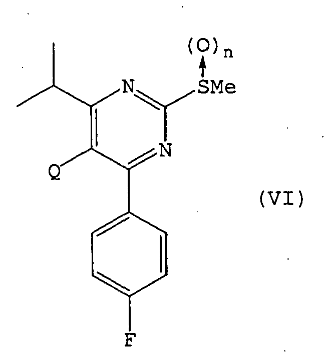 wherein n is 1 or 2, and Q is as defined above (hereinafter sometimes to be referred to as compound (VI)) or a salt thereof; and
   reacting the obtained compound (VI) or a salt thereof with N-methyl-methanesulfonamide.
[8] The production method of the above-mentioned [7], wherein Q is a methoxycarbonyl group.
[9] The production method of the above-mentioned [2], wherein X is a methylamino group, R¹ is an isopropyl group, and R² is a 4-fluorophenyl group.
[10] A production method of compound (VII) or a salt thereof, which comprises reacting a compound represented by the formula (I") : 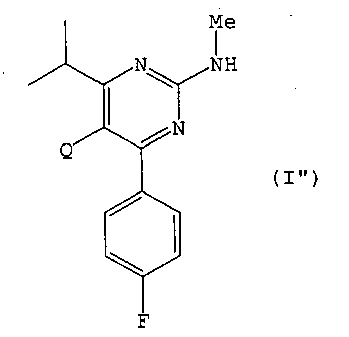 wherein Q is a carboxylate group (hereinafter sometimes to be referred to as compound (I")), which is obtained by the production method of the above-mentioned [9], or a salt thereof with methanesulfonyl chloride.
[11] The production method of the above-mentioned [10], wherein Q is a methoxycarbonyl group.
[12] A compound represented by the formula (IV): 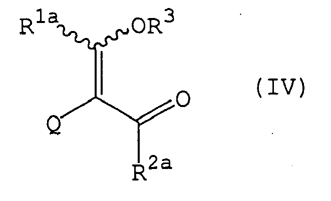 wherein R^{1a} and R^{2a} are the same or different and each is a lower alkyl group optionally having substituent(s), an aralkyl group optionally having substituent(s) or an aryl group optionally having substituent(s), R³ is a lower alkyl group, Q is a carboxylate group, and the wavy line shows a trans-isomer, a cis-isomer or a mixture thereof, excluding a compound wherein R^{1a}, R^{2a} and R³ are methyl groups and Q is a methoxycarbonyl group (hereinafter sometimes to be referred to as compound (IV)).
[13] The compound of the above-mentioned [12], wherein one of R^{1a} and R^{2a} is an isopropyl group, and the other is a 4-fluorophenyl group.
[14] A compound represented by the formula (VI'): 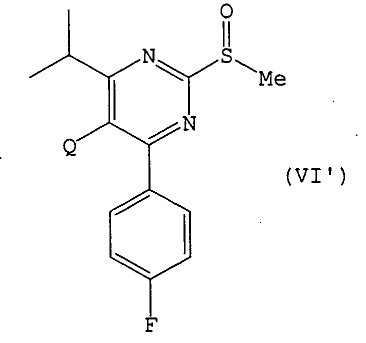 wherein Q is a carboxylate group (hereinafter sometimes to be referred to as compound (VI')) or a salt thereof.
[15] The compound of the above-mentioned [14], wherein Q is a methoxycarbonyl group or a salt thereof.

### Detailed Description of the Invention

The present invention is explained in detail in the following.

### 1. Explanation of symbols

The "lower alkyl group" for R³ is a linear or branched chain alkyl preferably having 1 - 6, more preferably 1 or 2, carbon atoms and, for example, methyl group, ethyl group, n-propyl group, isopropyl group and the like can be mentioned. Of these, methyl group and ethyl group are preferable.

The "lower alkyl group optionally having substituent(s)" for R¹, R², R⁴, R⁵, R⁶, R⁷ or X is the above-mentioned alkyl group optionally substituted by one or more of the following substituents, and when the number of the substituents is two or more, they may be the same or different. Examples of the substituent include nitro group, linear or branched chain alkoxy group (preferable carbon atoms: 1 - 6, e.g., methoxy group and the like), halogen atom (e.g., chlorine atom, fluorine atom and the like), hydroxyl group and the like.

The "aryl" of the "aryl group optionally having substituent(s)" for R¹, R², R⁴, R⁵, R⁶, R⁷ and X is an aryl group having preferably 6 - 10, carbon atoms and, for example, phenyl group, 1-naphthyl group, 2-naphthyl group and the like can be mentioned. The aryl group may be substituted by one or more substituents below. When the number of the substituents is two or more, they may be the same or different. Examples of the substituent include nitro group, linear or branched chain alkoxy group (preferable carbon atoms: 1 - 6, e.g., methoxy group and the like), halogen atom (e.g., chlorine atom, fluorine atom and the like), linear or branched chain alkyl group (preferable carbon atoms: 1 - 4, e.g., methyl group, ethyl group, propyl group and the like), hydroxyl group and the like.

The "aralkyl group" of the "aralkyl group optionally having substituent(s)" for R¹, R², R⁵, R⁶ and X is aralkyl group wherein the aryl moiety is aryl group preferably having 6 - 10, more preferably 6, carbon atoms and the alkyl moiety is linear or branched chain alkyl group preferably having 1 - 6 carbon atoms. For example, benzyl group and the like can be mentioned.

The aralkyl group may be substituted by one or more substituents below. When the number of the substituents is two or more, they may be the same or different. Examples of the substituent include nitro group, linear or branched chain alkoxy group (preferable carbon atoms: 1 - 6, e.g., methoxy group and the like), halogen atom (e.g., chlorine atom, fluorine atom and the like), linear or branched chain alkyl group (preferable carbon atoms: 1 - 4, e.g., methyl group, ethyl group, propyl group and the like), hydroxyl group and the like.

Examples of the "aliphatic hetero ring" optionally formed by R⁵ and R⁶ together with the adjacent nitrogen atom include 5- or 6-membered aliphatic hetero ring containing a carbon atom and at least one nitrogen atom and also optionally containing 1 - 3 hetero atoms selected from oxygen atom, sulfur atom and nitrogen atom, such as pyrrolidine, piperidine, morpholine, thiomorpholine, piperazine and the like.

The aliphatic hetero ring may be substituted by one or more substituents below. When the number of the substituents is two or more, they may be the same or different. Examples of the substituent include nitro group, linear or branched chain alkoxy group (carbon atoms: 1 - 6, e.g., methoxy group and the like), halogen atom (e.g., chlorine atom, fluorine atom and the like), linear or branched chain alkyl group (preferable carbon atoms: 1 - 4, e.g., methyl group, ethyl group, propyl group and the like), hydroxyl group and the like.

Examples of the carboxylate group represented by Q include methoxycarbonyl group, ethoxycarbonyl group, propoxycarbonyl group, tert-butoxycarbonyl group, benzyloxycarbonyl group and the like. A methoxycarbonyl group and an ethoxycarbonyl group are preferable.

Now, preferable embodiments of each symbol are explained below.

As the X, hydrogen atom, methyl group, phenyl group, methoxy group, methylthio group, benzylthio group, amino group, methylamino group or N-methanesulfonyl-N-methylamino group is preferable, and hydrogen atom, methylthio group or methylamino group is more preferable.

As the R¹, methyl group or isopropyl group is preferable.

As the R², methyl group or aryl group optionally having substituent(s) is preferable, and methyl group, 4-fluorophenyl group or 2,4-dimethylphenyl group is more preferable.

Compound (I), (I'), (I"), (VI), (VI') and (VII) have a pyrimidine ring, and can form a salt such as an inorganic acid salt (e.g., hydrochloride, sulfate and the like), an organic acid salt (e.g., acetate, trifluoroacetate, tosylate, mesylate and the like) and the like.

As compound (V), those generally commercially available in the form of a salt such as an inorganic acid salt (e.g., hydrochloride, sulfate and the like), an organic acid salt (e.g., acetate, trifluoroacetate, tosylate, mesylate and the like) and the like can be used.

Compound (II) is generally present as a tautomer mixture of enol forms represented by the following formulas (IIa) and (IIb) (hereinafter to be also referred to as compound (IIa) and compound (IIb), respectively). 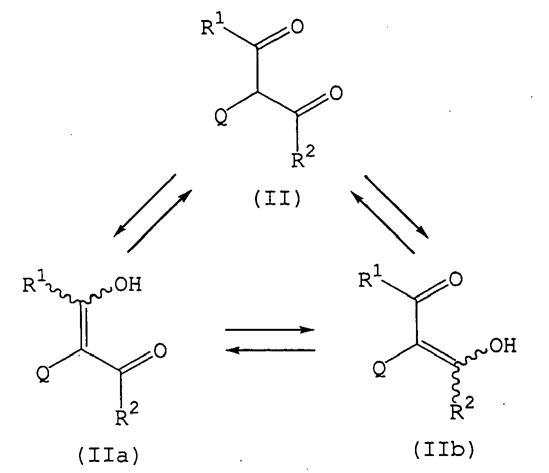 wherein each symbol and the wavy line are as defined above.

In the present invention, a simple reference to compound (II) means compound (II), compound (IIa), compound (IIb) or even a mixture thereof.

### 2. Production method of compound (I)

The production method of the present invention is characterized in that it contains the following step (a) shown by the following reaction scheme. 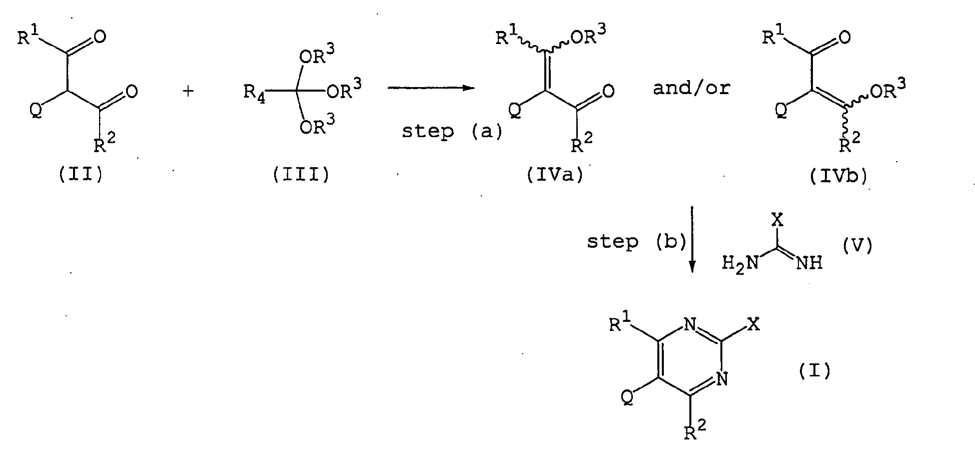 wherein each symbol and the wavy line are as defined above.

The pyrimidine ring can be constructed in a high yield by employing economical and highly safe ortho ester (III) as an enolation agent in such synthetic route, particularly step (a).

Steps (a) - (b) are explained below.

### 2-1. Step (a)

Step (a) can be performed, for example, by mixing compound (II) and ortho ester (III) in a solvent or without a solvent. The order of addition of respective reagents is not particularly limited and they can be added successively or simultaneously.

As the ortho ester (III) used in step (a), a commercially available product is preferably used and, for example, trimethyl orthoformate, triethyl orthoformate, trimethyl orthoacetate, triethyl orthoacetate, methyl orthopropionate, triethyl orthopropionate, trimethyl orthobutyrate, triethyl orthobutyrate, trimethyl orthobenzoate and the like, particularly, trimethyl orthoacetate, trimethyl orthobenzoate, trimethyl orthobutyrate and triethyl orthobutyrate are preferable.

The amount of orthoester (III) to be used is generally 0.8 - 30 equivalents, preferably 1 - 5 equivalents, relative to compound (II). When the amount of orthoester (III) is smaller than this range, the reaction tends to proceed slowly.

For step (a), an acid is preferably added as a catalyst to promote the reaction. Examples of the acid include sulfuric acid, methanesulfonic acid, toluenesulfonic acid and the like, with preference given to sulfuric acid, methanesulfonic acid and toluenesulfonic acid. The amount of the acid to be used is generally 0.001 - 0.1 equivalent, preferably 0.005 - 0.05 equivalent, relative to compound (II). When the amount of the acid to be used is smaller than this range, the reaction tends to proceed slowly.

In step (a), a drying agent is preferably added to remove a trace amount of water contained in the reaction mixture. Examples of the drying agent include magnesium sulfate, molecular sieve, sodium sulfate and the like, with preference given to magnesium sulfate and molecular sieve. The amount of the drying agent to be used is generally 0.1-fold weight to 20-fold weight, preferably 0.2-fold weight to 2-fold weight, relative to compound (II). When the amount of the drying agent to be used is smaller than this range, the yield tends to decrease.

While step (a) may be performed in a solvent, it is preferably performed without a solvent. When a solvent is used, the solvent may be any as long as it does not inhibit this reaction, for example, hydrocarbon solvent (e.g., toluene, benzene and the like) and the like can be mentioned, which may be used alone or in a combination of two or more kinds thereof, and toluene is preferable. The amount of the solvent to be used is 1-fold weight to 30-fold weight, preferably 5-fold weight to 20-fold weight, relative to compound (II).

Step (a) is generally performed within the range of from 50°C to the reflux temperature of the solvent or the ortho ester (III) to be used (preferably 60°C to 150°C). The reaction time is generally 0.5 hr - overnight (preferably 1 hr - 8 hr) within the above-mentioned temperature range.

After the completion of step (a), the reaction mixture contains one of compound (IVa) and compound (IVb), or a mixture thereof. However, a mixture of compound (IVa) and compound (IVb) can be subjected to step (b) without particular separation.

Compound (IVa) and/or compound (IVb) can be isolated and purified by a conventional method. Compound (IVa) and/or compound (IVb) can be isolated by, after completion of the reaction, where necessary, extracting with ethyl acetate, methyl-butyl-ether etc., washing with water, aqueous acidic solution (e.g., hydrochloric acid, sulfuric acid and the like) or aqueous alkali solution (e.g., saturated aqueous sodium hydrogen carbonate, brine and the like) and the like, and concentrating the organic layer obtained by partitioning. Moreover, compound (IVa) and/or compound (IVb) can be purified by, but is not limited to, performing crystallization by adding a crystallization solvent (e.g., ethers (e.g., diethyl ether, THF and the like), acetone, acetonitrile, hydrocarbon solvent (e.g., toluene, benzene, hexane, heptane and the like), a halogen solvent (e.g., dichloromethane, dichloroethane and the like), alcohols (e.g., methanol, ethanol, isopropanol and the like), water, a mixed solvent thereof and the like), silica gel column chromatography or distillation. In addition, compound (IVa) and/or compound (IVb) can be subjected to the next step without purification.

Compound (II), which is a starting material for step (a), can be produced by a known method (e.g., JP-A-6-256318), or a commercially available product can also be used.

Compound (IVa) and compound (IVb) obtained in step (a), excluding a compound wherein R¹, R² and R³ are methyl groups and Q is a methoxycarbonyl group, namely, compound (IV), is a novel compound, and is a useful synthetic intermediate for various pharmaceutical products.

Compound (IV) wherein one of R^{1a} and R^{2a} is an isopropyl group and the other is a 4-fluorophenyl group is useful as an intermediate for Crestor, a cholesterol lowering agent.

### 2-3. step (b)

Step (b) can be performed, for example, by mixing compound (IVa) and/or compound (IVb) and compound (V) in a solvent. The order of addition of respective reagents is not particularly limited and they can be added successively or simultaneously.

Compound (V) used in step (b) is generally in the form of a commercially available stable salt. As the salt of compound (V), for example, acid addition salts such as hydrochloride, sulfate, acetate and the like can be mentioned.

When an acid addition salt of compound (V) is to be used in step (b), it can be neutralized with a base in a solvent. In this case, for example, the salt is once converted to a free form, followed by reaction with compound (IVa) and/or compound (IVb). Alternatively, for example, a salt of compound (V) and compound (IVa) and/or compound (IVb) are dissolved in a solvent, followed by addition of a base to allow reaction. The base to be used for neutralization is not particularly limited and, for example, sodium hydroxide, potassium hydroxide, sodium carbonate, potassium carbonate, triethylamine, sodium methoxide, sodium ethoxide and the like can be used. The amount of the base to be used is not particularly limited as long as it can convert the salt of compound (V) to a free form. From the economical viewpoint, it is generally 0.8 - 3 equivalents, preferably 1 - 1.5 equivalents, relative to the salt of compound (V).

The solvent usable for step (b) may be any as long as it does not inhibit the reaction and, for example, esters (e.g., ethyl acetate, propyl acetate, butyl acetate and the like), nitriles (e.g., acetonitrile and the like), alcohols (e.g., methanol, ethanol, isopropyl alcohol and the like) and the like can be mentioned, which may be used alone or in a combination of two or more kinds thereof, and acetonitrile, ethyl acetate or ethanol is preferable. The amount of the solvent to be used is 2-fold weight to 30-fold weight, preferably 5-fold weight to 20-fold weight, relative to compound (IVa) and/or compound (IVb).

Step (b) is generally performed within the range of from 0°C to the reflux temperature of the solvent to be used (preferably 40°C to 100°C). The reaction time is generally 0.5 hr - 30 hr (preferably 1 hr - 20 hr) within the above-mentioned temperature range.

Compound (I) obtained in step (b) can be isolated and purified by a conventional method. For example, compound (I) can be isolated by, after completion of the reaction, where necessary, extracting with ethyl acetate etc., washing with water, aqueous acidic solution (e.g., hydrochloric acid, sulfuric acid and the like), aqueous alkali solution (e.g., saturated aqueous sodium hydrogen carbonate and the like) or brine and the like, and concentrating the organic layer obtained by partitioning. Moreover, compound (I) can be purified by, but is not limited to, performing crystallization by adding a crystallization solvent (e.g., ethers (e.g., diethyl ether, THF and the like), acetone, acetonitrile, hydrocarbon solvent (e.g., toluene, benzene, hexane, heptane and the like), a halogen solvent (e.g., dichloromethane, dichloroethane and the like), alcohols (e.g., methanol, ethanol, isopropanol and the like), water, a mixed solvent thereof and the like), or silica gel column chromatography. In addition, compound (I) can be used as a synthetic intermediate without purification.

### 3. Production method of compound (VII)

Compound (VII) is known to be useful as an important synthetic intermediate for Crestor, a cholesterol lowering agent having HMGCoA reductase inhibitory activity.

Compound (VII) can be produced from compound (I'), among compound (I) produced by the present invention, as a starting material by the method described in JP-A-5-178841 shown in the following scheme or a method analogous thereto. 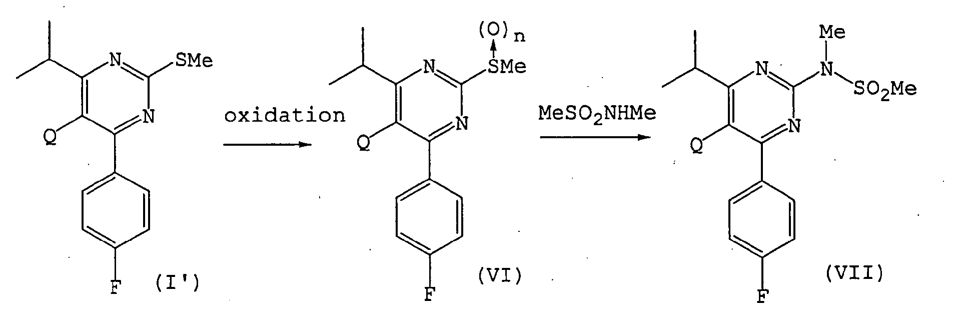 wherein Q and n are as defined above.

Compound (VI) wherein n is 1, namely, compound (VI'), is a novel compound, which requires a smaller amount of the oxidant to be used as compared to compound wherein n is 2, and is economical.

Compound (VII) can also be produced from compound (I") as a starting material by the method described in JP-A-6-256318 shown in the following scheme. 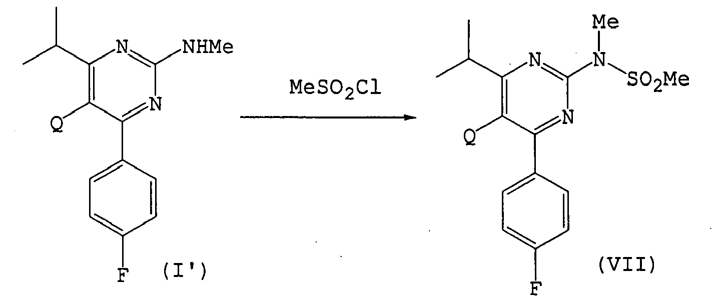 wherein Q is as defined above.

### Examples

The present invention is explained in more detail in the following by referring to Examples, which are not to be construed as limitative. The starting material, methyl-3-(4-fluorophenyl)-3-hydroxy-2-isobutyryl-propenoate, was obtained in the same manner as in Example 1 of JP-A-6-256318.

### Example 1 : mixture of methyl-2-(4-fluorobenzoyl)-3-methoxy-3-isopropylpropenoate and methyl-3-(4-fluorophenyl)-3-methoxy-2-isobutyryl-propenoate

To methyl-3-(4-fluorophenyl)-3-hydroxy-2-isobutyrylpropenoate (1.0 g, 3.75 mmol) were added trimethyl orthobutyrate (2.3 g, 15.5 mmol), magnesium sulfate (300 mg) and sulfuric acid (10 mg), and the mixture was heated under reflux for 3 hr. Methyl-tert-butyl-ether (MTBE) was added, the precipitate was filtrated, and the filtrate was concentrated under reduced pressure. The residue was purified by silica gel chromatography to give a regioisomer mixture of the title compound. It was detected as a regioisomer mixture in NMR spectrum.
¹H-NMR(CDCl₃) δ 0.93(1.5H, d, J=6.6Hz), 1.20(4.5H, d, J=6.9Hz), 2.36-2.46(0.25H, m), 2.97-3.05(0.75H, m), 3.46(2.25H, s), 3.52(2.25H, s), 3.55(0.75H, s), 3.82(0.75H, s), 7.12-7.16(2H, m), 7.34-7.37(2H, s)

### Example 2: methyl-2-(4-fluorobenzoyl)-3-methoxy-3-isopropylpropenoate

To methyl-3-(4-fluorophenyl)-3-hydroxy-2-isobutyrylpropenoate (100 mg, 0.38 mmol) were added trimethyl orthobutyrate (1.0 g, 6.75 mmol), a molecular sieve (100 mg) and sulfuric acid (2 µl), and the mixture was heated at 140°C for 1 hr. After cooling, MTBE was added and the precipitate was filtrated. The filtrate was analyzed by HPLC and confirmed to contain methyl-2-(4-fluorobenzoyl)-3-methoxy-3-isopropylpropenoate (85.3 mg, 0.11 mmol).
HPLC analysis conditions:
column: Inertsil ODS-2 4.6 mm×150_{φ}
column temperature: 40°C
detection wavelength: 254 nm
mobile phase: Buffer (NaH₂PO₄, pH 4.5):CH₃CN=40:60
flow rate: 0.8 ml/min.

### Example 3: methyl-2-(4-fluorobenzoyl)-3-methoxy-3-isopropylpropenoate

To methyl-3-(4-fluorophenyl)-3-hydroxy-2-isobutyrylpropenoate (100 mg, 0.38 mmol) were added trimethyl orthobutyrate (1.0 g, 6.75 mmol) and magnesium sulfate (100 mg), and the mixture was heated to 140°C and stirred for 1 hr. After cooling, MTBE was added, and the precipitate was filtrated. The filtrate was analyzed by HPLC under the same conditions as in Example 2 and confirmed to contain the title compound (83.2 mg, 0.30 mmol).

### Example 4: methyl-2-(4-fluorobenzoyl)-3-methoxy-3-isopropylpropenoate

To methyl-3-(4-fluorophenyl)-3-hydroxy-2-isobutyrylpropenoate (100 mg, 0.38 mmol) were added trimethyl orthoacetate (1.0 g, 8.33 mmol), magnesium sulfate (100 mg) and sulfuric acid (2 µl), and the mixture was heated to 140°C and stirred for 1 hr. The reaction mixture was analyzed by HPLC under the same conditions as in Example 2 and confirmed to contain the title compound (60.1 mg, 0.21 mmol).

### Example 5: methyl-2-(4-fluorobenzoyl)-3-methoxy-3-isopropylpropenoate

To methyl-3-(4-fluorophenyl)-3-hydroxy-2-isobutyrylpropenoate (100 mg, 0.38 mmol) were added trimethyl orthobenzoate (1.0 g, 5.49 mmol), magnesium sulfate (100 mg) and sulfuric acid (2 µl), and the mixture was heated to 140°C and stirred for 1 hr. The reaction mixture was analyzed by HPLC under the same conditions as in Example 2 and confirmed to contain the title compound (53.7 mg, 0.19 mmol).

### Example 6: ethyl-2-(1-ethoxyethylidene)-acetoacetate

To ethyl-diacetoacetate (510 mg, 2.96 mmol) were added triethyl orthobutyrate (845 mg, 4.39 mmol), magnesium sulfate (200 mg) and sulfuric acid (5 µl), and the mixture was heated at 140°C for 6 hr. MTBE was added, and the precipitate was filtrated. The filtrate was concentrated under reduced pressure, and the residue was purified by silica gel column chromatography to give an oil containing ethyl-2-(1-ethoxyethylidene)-acetoacetate (449 mg, 2.24 mmol).
¹H-NMR(CDCl₃) δ 1.21(6H, m), 2.18(1.2H, s), 2.34(3.6H, s), 2.43(1.2H, s), 4.04-4.10(2H, m), 4.17(2H, q, J=7.2Hz), 4.28(1H, q, J=7.2Hz)

### Example 7: methyl 4-(4-fluorophenyl)-6-isopropyl-2-methylthiopyrimidine-5-carboxylate

Methylisothiourea sulfate (3.72 g, 13.36 mmol) and sodium carbonate (3.03 g, 28.55 mmol) were added to acetonitrile (50 ml), and the mixture was stirred at room temperature for 30 min. Methyl-2-(4-fluorobenzoyl)-3-methoxy-3-isopropylpropenoate (5.00 g, 17.83 mmol) was added, and the mixture was stirred at 80°C overnight. The reaction mixture was concentrated, ethyl acetate (60 ml) was added, and the mixture was washed successively with water and saturated brine. The organic layer was dried over sodium sulfate, and the drying agent was filtered off. The filtrate was concentrated to dryness, and the residue was purified by silica gel column chromatography (hexane-ethyl acetate) to give methyl 4-(4-fluorophenyl)-6-isopropyl-2-methylthiopyrimidine-5-carboxylate (4.82 g, 15.04 mmol).
¹H-NMR(CDCl₃) δ 1.31(6H, d, J=6.7Hz), 2.61 (1H, s), 3.17 (1H, hep, J=6.7Hz), 3.70(3H, s), 7.10-7.15(2H, m), 7.62-67(2H, m)

### Example 8: methyl 4-(4-fluorophenyl)-6-isopropyl-2-methylaminopyrimidine-5-carboxylate

1-Methylguanidine hydrochloride (391 mg, 3.57 mmol) and sodium carbonate (378 mg, 3.57 mmol) were added to acetonitrile (8 ml). After stirring the mixture at room temperature for 30 min, methyl-2-(4-fluorobenzoyl)-3-methoxy-3-isopropylpropenoate (500 mg, 1.78 mmol) was added, and the mixture was stirred at 80°C overnight. The reaction mixture was analyzed by HPLC under the same conditions as in Example 2, and the production of methyl 4-(4-fluorophenyl)-6-isopropyl-2-methylaminopyrimidine-5-carboxylate (448 mg, 1.48 mmol) was confirmed. By a treatment in the same manner as in Example 7, the title compound was obtained.
¹H-NMR(CDCl₃) δ 1.26(6H, d, J=6.6Hz), 3.05(3H, d, J=5.1Hz), 3.15(1H, hep, J=6.6Hz), 3.61(3H, s), 5.30(1H, br), 7.08-7.12(2H, m), 7.52-7.60(2H, m)

### Example 9: ethyl 4,6-methyl-2-methylthiopyrimidine-5-carboxylate

Methylisothiourea sulfate (40 mg, 0.14 mmol) and sodium carbonate (32 mg, 0.30 mmol) were suspended in acetonitrile (1 ml), ethyl-2-(1-ethoxyethylidene)-acetoacetate (60 mg, 0.30 mmol) was added, and the mixture was stirred at 80°C overnight. The reaction mixture was cooled, and washed successively with water and saturated brine. After concentration under reduced pressure, the residue was purified by silica gel chromatography to give the title compound (46 mg, 0.20 mmol).
¹H-NMR(CDCl₃) δ 1.40(3H, t, J=7.1Hz), 2.50(3H, s), 2.57(3H, s), 4.20(2H, q, J=7.1Hz)

### Example 10: ethyl 4,6-dimethylpyrimidine-5-carboxylate

To a suspension of formamidine acetate (100 mg, 0.96 mmol) in ethanol (1 ml) was added a solution (331 mg) of 21% sodium ethoxide in ethanol. Ethyl-2-(1-ethoxyethylidene)-acetoacetate (192 mg, 0.96 mmol) was added, and the mixture was stirred at 80°C for 3 hrs. After concentration under reduced pressure, the residue was purified by silica gel chromatography to give the title compound (78 mg, 0.43 mmol).
¹H-NMR(CDCl₃) δ 1.42(3H, t, J=7.2Hz), 2.55(6H, s), 4.47(2H, q, J=7.2Hz), 9.02(1H, s)

### Example 11: ethyl 4,6-methyl-2-methylthiopyrimidine-5-carboxylate

To ethyl diacetoacetate (510 mg, 2.96 mmol) were added trimethyl orthobutyrate (845 mg, 4.39 mmol), magnesium sulfate (200 mg) and sulfuric acid (5 µl), and the mixture was stirred at 140°C for 4 hr in an oil bath. The reaction mixture was concentrated under reduced pressure. The residue was added to a suspension of methylisothiourea sulfate (412 mg, 1.48 mmol) and sodium carbonate (320 mg, 3.01 mmol) in ethyl acetate, and the mixture was stirred at 80°C overnight. After cooling, the mixture was washed successively with water and saturated brine, and concentrated under reduced pressure. The residue was purified by silica gel chromatography to give the title compound (365 mg, 1.61 mmol).

### Example 12: methyl 4-(4-fluorophenyl)-6-isopropyl-2-methylthiopyrimidine-5-carboxylate

To methyl 3-(4-fluorophenyl)-3-hydroxy-2-isobutyrylpropenoate (1.0 g, 3.75 mmol) were added trimethyl orthobutyrate (1.0 g, 6.75 mmol), magnesium sulfate (400 mg) and sulfuric acid (10 mg), and the mixture was stirred at 140°C for 3 hr in an oil bath. The reaction mixture was concentrated. The residue was added to a suspension of methylisothiourea sulfate (520 mg, 1.87 mmol) and sodium carbonate (400 mg, 3.77 mmol) in ethyl acetate, and the mixture was stirred at 80°C overnight. After the reaction, the reaction mixture was washed successively with water and saturated brine. The organic layer was dried over sodium sulfate, filtrated, and the filtrate was concentrated. Methanol was added to the residue and the mixture was stirred. The precipitate was filtrated, and dried under reduced pressure to give methyl 4-(4-fluorophenyl)-6-isopropyl-2-methylthiopyrimidine-5-carboxylate (747 mg, 2.33 mmol).

### Example 13: methyl 4-(4-fluorophenyl)-6-isopropyl-2-methylsulfonylpyrimidine-5-carboxylate

Methyl 4-(4-fluorophenyl)-6-isopropyl-2-methylthiopyrimidine-5-carboxylate (1.50 g, 4.68 mmol) was dissolved in dichloromethane (30 ml), m-chloroperbenzoic acid (2.23 g, 11.70 mmol) was added under ice-cooling, and the mixture was stirred at room temperature overnight. Water (10 ml) was added to the reaction mixture, and the mixture was filtrated to separate the layers. The organic layer was washed with saturated aqueous sodium hydrogen carbonate and saturated brine. The organic layer was concentrated to dryness to give the title compound (1.59 g, 4.53 mmol).
¹H-NMR(CDCl₃) δ 1.37(6H, d, J=6.7Hz), 3.21(1H, hep, J=6.7Hz), 3.42 (1H, s), 3.81(3H, s), 7.16-7.20(2H, m), 7.74-7.78(2H, m)

### Example 14: methyl 4-(4-fluorophenyl)-6-isopropyl-2-(N-methanesulfonyl-N-methylamino)-pyrimidine-5-carboxylate

Methyl 4-(4-fluorophenyl)-6-isopropyl-2-methylsulfonylpyrimidine-5-carboxylate (1.59 g, 4.53 mmol) was dissolved in butyl acetate, potassium carbonate (776 mg, 5.61 mmol) and methylmethanesulfonamide (564 mg, 5.16 mmol) were added, and the mixture was stirred at 90°C for 3 hr. The reaction mixture was washed with water and saturated brine. The organic layer was concentrated, and crystallized from methanol. The obtained crystals were filtrated and dried to give the title compound (1.68 g, 4.40 mmol).
¹H-NMR(CDCl₃) δ 1.31(6H, d, J=6.7Hz), 3.18(1H, hep, J=6.7Hz), 3.52(1H, s), 3.60(3H, s), 3.69(3H, s), 7.10-7.16(2H, m), 7.64-7.70(2H, m)

### Example 15: methyl 4-(4-fluorophenyl)-6-isopropyl-2-methylsulfinylpyrimidine-5-carboxylate

Methyl 4-(4-fluorophenyl)-6-isopropyl-2-methylthiopyrimidine-5-carboxylate (157 mg, 0.49 mmol) was dissolved in chloroform (2 ml), a solution of m-chloroperbenzoic acid (2.23 g, 0.65 mmol) in dichloromethane was added dropwise under ice-cooling over 30 min, and the mixture was stirred at room temperature for 2 hr. The reaction mixture was washed successively with water, saturated aqueous sodium hydrogencarbonate and saturated brine. The organic layer was concentrated to dryness to give the title compound (156 mg, 0.46 mmol).
¹H-NMR(CDCl₃) δ 1.37 (6H, d, J=6.6Hz), 3.05 (1H, s), 3.20 (1H, hep, J=6.6Hz), 3.79(3H, s), 7.10-7.17(2H, m), 7.74-7.78(2H, m)

### Example 16: methyl 4-(4-fluorophenyl)-6-isopropyl-2-(N-methanesulfonyl-N-methylamino)-pyrimidine-5-carboxylate

Methyl 4-(4-fluorophenyl)-6-isopropyl-2-methylsulfinylpyrimidine-5-carboxylate (156 mg, 0.46 mmol) was dissolved in butyl acetate, potassium carbonate (70 mg, 0.51 mmol) and methylmethanesulfonamide (55 mg, 0.50 mmol) were added, and the mixture was stirred at 90°C overnight. The reaction mixture was washed with water and saturated brine. The organic layer was concentrated and methanol was added. The crystals were filtrated and dried to give the title compound (146 mg, 0.39 mmol).

### Example 17: methyl 4-(4-fluorophenyl)-6-isopropyl-2-(N-methanesulfonyl-N-methylamino)-pyrimidine-5-carboxylate

Methyl 4-(4-fluorophenyl)-6-isopropyl-2-methylaminopyrimidine-5-carboxylate (100 mg, 0.33 mmol) was dissolved in DMF (2 ml), 55% NaH (16 mg, 0.35 mmol) was added, and the mixture was stirred at room temperature for 15 min. Methanesulfonyl chloride (34 µl) was added, and the mixture was stirred for 1 hr. The reaction mixture was concentrated and purified by silica gel chromatography to give the title compound (78 mg, 0.20 mmol).

### Comparative Example 1: methyl-2-(4-fluorobenzoyl)-3-methoxy-3-isopropylpropenoate (byproduction of 2-(4-fluorobenzoyl)-2,4-dimethyl-3-oxo-pentanoate)

Methyl 3-(4-fluorophenyl)-3-hydroxy-2-isobutyrylpropenoate (2.25 g, 8.5 mmol) was dissolved in acetonitrile (30 ml), sodium carbonate (1.35 g, 12.8 mmol) and methyl sulfate (1.18 g, 9.4 mmol) were added, and the mixture was stirred at 80°C overnight. The reaction mixture was concentrated, ethyl acetate was added, and the mixture was washed successively with water and saturated brine. The organic layer was concentrated, and the residue was purified by silica gel chromatography to give methyl 2-(4-fluorobenzoyl)-3-methoxy-3-isopropylpropenoate (0.80 g, 2.9 mmol). As a byproduct, C-methylated form, methyl 2-(4-fluorobenzoyl)-2,4-dimethyl-3-oxo-pentanoate (0.61 mg, 2.2 mmol), was also isolated.

### Comparative Example 2: methyl-2-(4-fluorobenzoyl)-3-methoxy-3-isopropylpropenoate (byproduction of 2-(4-fluorobenzoyl)-2,4-dimethyl-3-oxo-pentanoate)

Methyl 3-(4-fluorophenyl)-3-hydroxy-2-isobutyrylpropenoate (92 mg, 0.35 mmol) was dissolved in acetonitrile (2 ml), and cesium carbonate (113 mg, 0.41 mmol) was added. Under ice-cooling, methyl trifluoromethanesulfonate (39 µl, 0.36 mmol) was added dropwise, and the mixture was stirred at room temperature for 3 hrs. The reaction mixture was analyzed by HPLC under the same conditions as in Example 2, and production of methyl 2-(4-fluorobenzoyl)-3-methoxy-3-isopropylpropenoate (37%) as well as a C-methylated form, methyl 2-(4-fluorobenzoyl)-2,4-dimethyl-3-oxo-pentanoate (45%), as a byproduct were confirmed.

### Industrial Applicability

According to the method of the present invention, use of ortho esters, which are economical and low toxic, in enol etherification of compound (II) suppresses C-alkylation, and affords compound (IVa) and/or compound (IVb) in a high yield. As a result, compound (I) and compound (VII) can be produced in a high yield.

This application is based on a patent application No. 2005-027065 filed in Japan, the contents of which are incorporated in full herein by this reference.

## Claims

1. A production method of a compound represented by the following formula (IVa) : 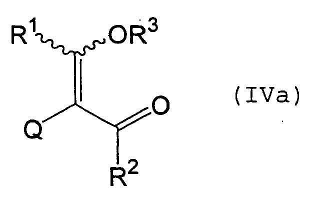 wherein R¹ and R² are the same or different and each is a lower alkyl group optionally having substituent(s), an aralkyl group optionally having substituent(s) or an aryl group optionally having substituent(s), R³ is a lower alkyl group, Q is a carboxylate group, and the wavy line is a trans-isomer, a cis-isomer or a mixture thereof and/or a compound represented by the formula (IVb): 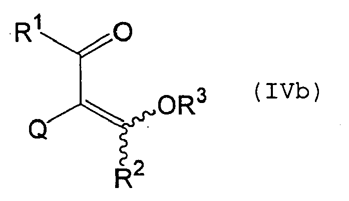 wherein the wavy line and each symbol are as defined above, which comprises reacting a compound represented by the formula (II) : 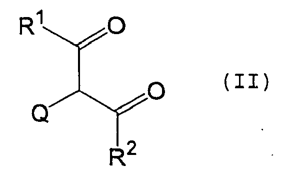 wherein each symbol is as defined above with an ortho ester represented by the formula (III): 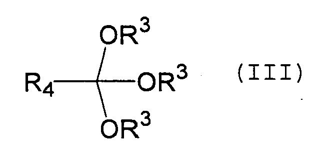 wherein R³ is as defined above, and R⁴ is a hydrogen atom, a lower alkyl group optionally having substituent(s) or an aryl group optionally having substituent(s).

2. A production method of a compound represented by the formula (I) : 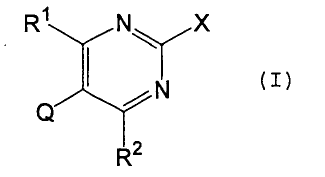 wherein X is a hydrogen atom, a lower alkyl group optionally having substituent(s), an aralkyl group optionally having substituent(s), an aryl group optionally having substituent(s), -OR⁵, -SR⁵ or -NR⁵R⁶ (wherein R⁵ and R⁶ are the same or different and each is a hydrogen atom, a lower alkyl group optionally having substituent(s), an aralkyl group optionally having substituent(s), an aryl group optionally having substituent(s) or -SO₂R⁷ (wherein R⁷ is a lower alkyl group optionally having substituent(s) or an aryl group optionally having substituent(s)), or R⁵ and R⁶ may form, together with the adjacent nitrogen atom, an aliphatic hetero ring optionally having substituent(s)), and other symbols are as defined in claim 1 or a salt thereof, which comprises reacting a compound represented by the following formula (IVa): 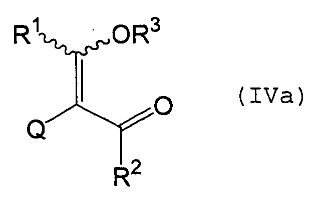 wherein the wavy line and each symbol are as defined in claim 1 and/or a compound represented by the formula (IVb): 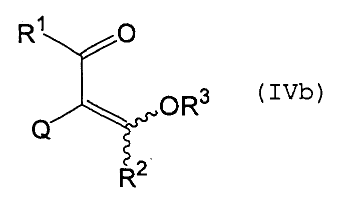 wherein the wavy line and each symbol are as defined in claim 1 obtained by the production method of claim 1, with a compound represented by the formula (V): or 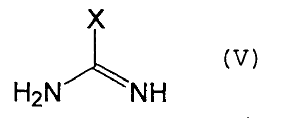 wherein X is as defined above or a salt thereof.

3. The production method of claim 2, wherein X is a hydrogen atom, a methyl group, a phenyl group, a methoxy group, a methylthio group, a benzylthio group, an amino group, a methylamino group or an N-methanesulfonyl-N-methylamino group.

4. The production method of any one of claims 1 to 3, wherein R¹ is a methyl group or an isopropyl group, and R² is a methyl group or an aryl group optionally having substituent(s).

5. The production method of any one of claims 1 to 4, wherein Q is a methoxycarbonyl group or an ethoxycarbonyl group.

6. The production method of claim 2, wherein X is a methylthio group, R¹ is an isopropyl group, and R² is a 4-fluorophenyl group.

7. A production method of a compound represented by the formula (VII): 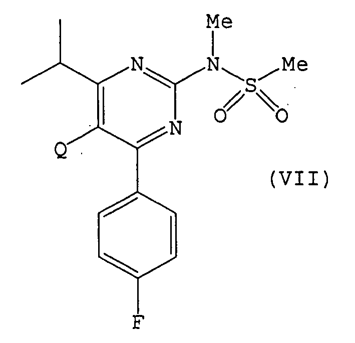 wherein Q is a carboxylate group or a salt thereof, which comprises oxidizing a compound represented by the formula (I'): 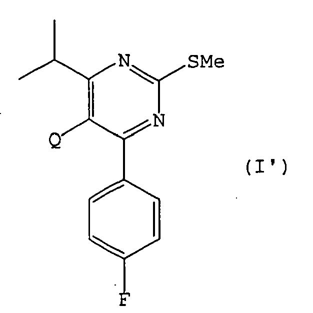 wherein Q is as defined above, which is obtained by the production method of claim 6, or a salt thereof to give a compound represented by the formula (VI): 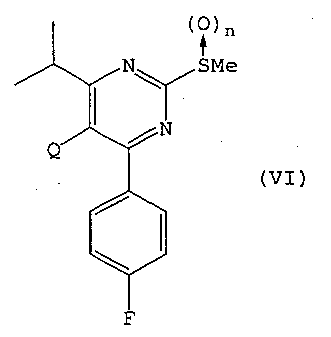 wherein n is 1 or 2, and Q is as defined above or a salt thereof; and
reacting the obtained compound represented by the formula (VI) or a salt thereof with N-methyl-methanesulfonamide.

8. The production method of claim 7, wherein Q is a methoxycarbonyl group.

9. The production method of claim 2, wherein X is a methylamino group, R¹ is an isopropyl group, and R² is a 4-fluorophenyl group.

10. A production method of a compound represented by the formula (VII): 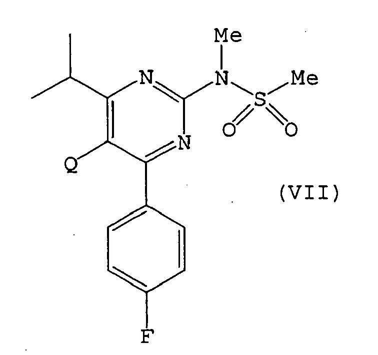 wherein Q is a carboxylate group or a salt thereof, which comprises reacting a compound represented by the formula (I") : 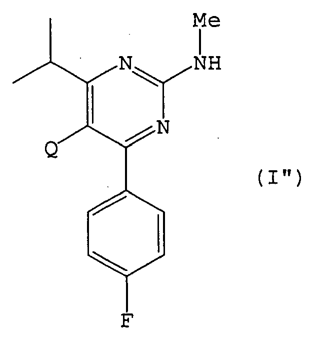 wherein Q is as defined above, which is obtained by the production method of claim 9, or a salt thereof with methanesulfonyl chloride.

11. The production method of claim 10, wherein Q is a methoxycarbonyl group.

12. A compound represented by the formula (IV): 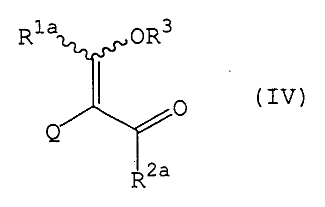 wherein R^{1a} and R^{2a} are the same or different and each is a lower alkyl group optionally having substituent(s), an aralkyl group optionally having substituent(s) or an aryl group optionally having substituent(s), R³ is a lower alkyl group, Q is a carboxylate group, and the wavy line shows a trans-isomer, a cis-isomer or a mixture thereof, excluding a compound wherein R^{1a}, R^{2a} and R³ are methyl groups and Q is a methoxycarbonyl group.

13. The compound of claim 12, wherein one of R^{1a} and R^{2a} is an isopropyl group, and the other is a 4-fluorophenyl group.

14. A compound represented by the formula (VI'): 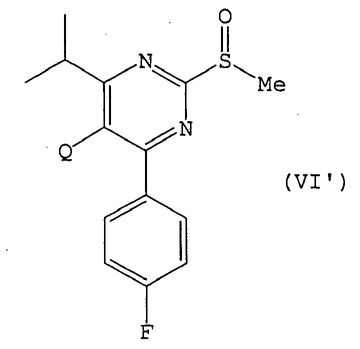 wherein Q is a carboxylate group or a salt thereof.

15. The compound of claim 14, wherein Q is a methoxycarbonyl group or a salt thereof.
